# EUROPEAN PATENT APPLICATION

(11) **EP 4 215 190 A1**
(43) Date of publication of application: **26.07.2023**
(21) Application number: 21868601.2
(22) Date of filing: 14.09.2021
(51) Int. Cl.: A61K 31/20, A61P 27/02, A61P 27/10

(54) **APPLICATION OF REGULATION OF EYE SCLERA LIPID METABOLISM TO INHIBIT MYOPIA**

(30) Priority: 17.09.2020 CN 202010976517; 27.05.2021 CN 202110586293; 10.09.2021 CN 202111065488
(71) Applicant: Wenzhou Medical University, Wenzhou, Zhejiang 325000 (CN)
(72) Inventor: ZHOU, Xiangtian, Wenzhou, Zhejiang 325000 (CN); PAN, Miaozhen, Wenzhou, Zhejiang 325000 (CN); ZHAO, Fei, Wenzhou, Zhejiang 325000 (CN); QU, Jia, Wenzhou, Zhejiang 325000 (CN)
(74) Representative: Cabinet Chaillot
(86) International application number: PCT/CN2021/118112
(87) International publication number: WO 2022/057778

(57) **Abstract**

The present invention relates to an application of inhibiting myopia by regulating eye scleral lipid metabolism. The present discloses a new mechanism leading to myopia, i.e., a close relationship between abnormal eye scleral lipid metabolism and myopia, thus revealing a new target for prevention and control of myopia; meanwhile, also provided is an eye drop that can effectively prevent and control myopia while avoiding eye allergies.

## Description

### CROSS-REFERENCE TO RELATED APPLICATION

The present application claims the priority of Chinese invention patent application CN202010976517.X filed on September 17, 2020. Moreover, the present application still claims the priority of Chinese invention patent application CN202110586293.6 filed on May 27, 2021. The present application still claims the priority of Chinese invention patent application CN202111065488.2 filed on September 10, 2021. CN202010976517. X, CN202110586293.6 and CN202111065488.2 are incorporated by reference herein in their entireties.

### TECHNICAL FIELD

The present invention relates to an application of inhibiting myopia by regulating eye scleral lipid metabolism, belonging to the field of biomedicines.

### BACKGROUND

Myopia is a common ametropia. The prevalence of myopia is now becoming an important public health problem in modern society (E. Dolgin, The myopia boom. Nature 519, 276-278 (2015); P. N. Baird et al., Myopia. Nature reviews. Disease primers 6, 99 (2020)). The proportion of myopic population in the world is predicted to increase from 28.3% (2010) to about 49.8% (2050) (B. A. Holden et al., Global Prevalence of Myopia and High Myopia and Temporal Trends from 2000 through 2050. Ophthalmology 123, 1036-1042 (2016)). In East Asia, this proportion may even reach 90%, of which up to 20% of myopia may be developed into high myopia (≥ 6.00 refraction [D]) (S. K. Jung, J. H. Lee, H. Kakizaki, D. Jee, Prevalence of myopia and its association with body stature and educational level in 19-year-old male conscripts in seoul, South Korea. Invest. Ophthalmol. Vis. Sci. 53, 5579-5583 (2012); I. Morgan, K. Rose, How genetic is school myopia? Prog. Retin. Eye Res. 24, 1-38 (2005)), which is one of the leading causes of irreversible blindness (J. Cooper, A. V. Tkatchenko, A Review of Current Concepts of the Etiology and Treatment of Myopia. Eye & contact lens 44, 231-247 (2018)).

There are varieties of myopia pathogenesis. Besides genetic factors, environmental factors, such as hypermodulation, peripheral retinal hyperopic defocus and form deprivation, also play a crucial role in onset and progression of myopia. At present, it is belived that the environmental factors are the major inducement of myopia prevalence, which affects the morbidity and severity of myopia, whereas genetically induced myopia cases are relatively rare. A specific mechanism by which environmental factors induce myopia may lie in that after the retina recognizes myopia-inducing visual information, these signals are transmitted via the choroid to the sclera, leading to changes in the extracellular matrix components of the sclera, which eventually causes the remodeling of the scleral extracellular matrix, leading to ocular axis elongation to develop myopia. Briefly, optical defocus triggers a defocus-specific signal that regulates the ocular refractive development (Wen-Yi Wang, Biomed Pharmacother. 2021 Jan; 133:111092.; Tatiana V Tkatchenko, Trends Pharmacol Sci. 2019 Nov;40(11):833-852.; and David Troilo, Invest Ophthalmol Vis Sci. 2019 Feb 28; 60(3):M31-M88.). The regulatory factors associated with the development of myopia described above include not only dopamine, choline, nitric oxide, retinoic acid, γ-aminobutyric acid (GABA), ZENK, nicotine, opiate and seroton, but also enzymes, proteoglycans and cytokines that directly regulate the collagen content, such as MMPs, TIMPs, SLRPs, FGF-2, IGF-1, TGF-β, insulin and glucagon, even some miRNAs are included (Wen-Yi Wang, Biomed Pharmacother. 2021 Jan;133:111092.). Myopia is mainly caused by the abnormal elongation of the vitreous chamber. This condition is reproduced by an animal model with monocular form deprivation (FD) to study the Occurrence and development of myopia. Eyeball elongation is associated with scleral tissue loss caused by sclera remodeling and reduced connective tissue synthesis, and changes in the composition and mechanical properties of the sclera due to increased type I collagen (COL1) degradation. Recent studies on monkeys have shown that the retina, particularly the photoreceptor and the retinal pigment epithelium (RPE), plays a crucial role in regulating eye growth and eye axis length by generating a signaling pathway that promotes scleral tissue remodeling. In fact, researchers have discussed and studied many causes that induce myopia, including the environmental factors mentioned above, such as extended book work or screen time and insufficient exposure to bright light, but there is still a need to focus on the study of additional possible mechanisms that cause myopia in order to provide new technical approaches for the treatment of myopia.

ω-3 polyunsaturated fatty acids are those in which a double bond farthest from a carboxyl group occurs on the last but two carbon atom, which are derived primarily from sea fish oil, such as squid oil, sardine oil, tuna oil, yellow tuna oil or fatty tuna oil. Substances belonging to ω-3 polyunsaturated fatty acids include but are not limited to: eicosapentaenoic acid (EPA), docosapentaenoic acid (DPA), docosahexaenoic acid (DHA), α-Lindenic acid (ALA). Studies have suggested that feeding EPA contributes to some alleviation of mouse myopia, however, the overall effect is not yet satisfactory, especially a first-pass effect occurs due to slow drug absorption by intragastric or oral administration, a dose reaching eyes is affected by a blood-eye barrier, resulting in great individual differences in drug efficacy, and there are clinical potential toxicity or side effects on other organs with high-dose administration, thus more optimal treatment schemes are urgently needed.

Researchers have already prepared ophthalmic drugs containing ω-3, such as eye drops, ophthalmic ointments, implantable tablets and ophthalmic gels, but subjects, after administration (e.g., by eye drops), are prone to developing allergic symptoms such as tearing, itching, swelling, upper and lower eyelid edema, mild lid conjunctival congestion, mild bulbar conjunctival congestion and edema, or flushing around the eye, thus limiting the practical application of such ophthalmic drugs.

In addition, considering that different ω-3 in the treatment of other diseases are significantly different in efficiency, there is a need to compare the efficiencies of different ω-3 on inhibiting the myopia-related risk factors, and an optimal scheme for treating myopia is provided by taking into account experimental results from multiple perspectives.

### SUMMARY OF THE INVENTION

The present invention solves at least four technical problems. First, an association mechanism between scleral lipid metabolism disorder and myopia is disclosed; second, the efficiencies of different ω-3 on inhibiting myopia-related risk factors and ocular axis elongation are investigated; third, allergic symptoms caused by eye application are eliminated while ensuring the safety of drugs (with little potential toxicity to other organs); and fourth, the optimal scheme for treating myopia with ω-3 is provided.

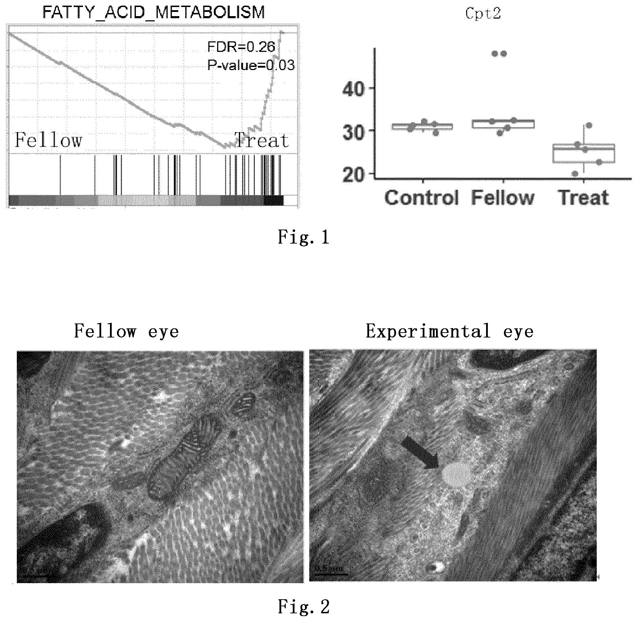

Specifically, in mouse transcriptome sequencing (RNA-seq) experiments, the inventors have surprisingly found that there are significant changes in the scleral lipid metabolic pathway in myopic eyes, especially downregulation in the expression of several key enzyme genes, such as carnitine palmitoyl transferase 2, in the scleral lipid metabolic pathway (FIG. 1). Moreover, lipid droplets are deposited in the sclera of myopic eyes as observed by electron microscopy (FIG. 2), further indicating scleral lipid metabolism disorder in myopic eyes. That indicates, the eye scleral lipid metabolism disorder or slow eye scleral lipid metabolism can be a cause of myopia, and therefore, a substance capable of regulating the eye scleral lipid metabolism may prevent, delay, inhibit, and/or treat myopia and myopia-related diseases.

It is known that there are many substances that can regulate eye scleral lipid metabolism, such as ω-3 polyunsaturated fatty acids, SREBP-1c inhibitors, and carnitine palmitoyl transferase 2 agonists. It is known that addition of endogenous omega-3 polyunsaturated fatty acids and supplementation of fish oil can reduce the transcription and protein expression of acute alcohol-induced upregulated SREBP-1c in liver. It is reported that SREBP-1c functions to promote lipid biosynthesis by upregulating the expression of adipogenesis-related genes, including ATP-citrate lyase (ACLY), acetyl coenzyme A carboxylase (ACC), fatty acid synthase (FAS), and stearoyl coenzyme A desaturase-1 (SCD-1). In the following specific embodiments, provided solely for the purposes of clear illustration of the scheme of treating myopia by regulating the eye scleral lipid metabolism of the present invention, the inventors have employed an ω-3 polyunsaturated fatty acid as an eye scleral lipid metabolism regulating substance to inhibit myopia, which should not instead be considered as a limitation to the scope of protection of the present invention.

Based on the above findings, the present invention provides a new target or a new mechanism for prevention and control of myopia, which can prevent and control myopia by regulating the level of the scleral lipid metabolism in individuals who are prone to myopia, individuals who are already myopic, or individuals who have a tendency to be myopic.

The present invention provides a method for regulating eye scleral lipid metabolism in an individual, and in particular relates to a method for delaying and inhibiting the development of myopia and myopia-related diseases by regulating the scleral lipid metabolism.

The present invention also provides the use of a scleral lipid metabolism signal pathway intervention regulator or a scleral lipid metabolism regulating substance as a drug for inhibiting the negative refraction, ocular axis elongation and/or vitreous chamber elongation of myopia eyes.

The present invention still provides the use of a ω-3 polyunsaturated fatty acid, an SREBP-1c inhibitor and/or a carnitine palmitoyl transferase 2 agonist in the manufacture of a preparation for reguling eye scleral lipid metabolism in an individual.

The present invention further provides a method for delaying and inhibiting myopia occurrence and development as well as myopia-related diseases by regulating scleral lipid metabolism.

The present invention further provides a method for preparing a pharmaceutical composition or a device for delaying and inhibiting myopia occurrence and development as well as treating myopia-related diseases by using an eye scleral lipid metabolism regulating substance.

The present invention provides a scleral lipid metabolism regulating composition which is made of the above mentioned substance. Preferably, the composition is a dietary supplement. More preferably, the composition is a ω-3 polyunsaturated fatty acid. Further, the dietary supplement comprises a flavoring agent, an antioxidant, a stabilizer and/or a preservative.

The present invention provides a method of inhibiting vitreous chamber elongation, ocular axis elongation or negative refraction of eyes preferably by applying a substance capable of regulating the eye scleral lipid metabolism pathway.

In one embodiment, the scleral lipid metabolism signal pathway intervention regulator or the scleral lipid metabolism regulating substance is ω-3. In one embodiment, the substance is not ω-3, preferably, the substance is an SREBP-1c inhibitor or a carnitine palmitoyl transferase 2 agonist.

The present invention also provides a method of regulating eye scleral lipid metabolism in an individual, wherein the ω-3 polyunsaturated fatty acid is administrated to the individual.

The present invention also provides a method of increasing choroidal blood perfusion in an individual, or a methodof increasing a choroidal thickness, or a method of inhibiting the increase of HIF-1α protein level, or a method of decreasing the HIF-1α protein level, or a method for increasing the expression level of carnitine palmitoyltransferase 2. In one embodiment, the ω-3 polyunsaturated fatty acid is administered to the individual.

The present invention also provides a method of increasing the choroidal thickness (ChT) of an individual and improving the choroidal blood perfusion (ChBP) of the individual to inhibit scleral hypoxia and its cascade reaction. In one embodiment, the ω-3 polyunsaturated fatty acid is administered to the individual.

The present invention also provides use of a substance capable of inhibiting the reduction in choroidal thickness (ChT), and relieving the decrease in choroidal blood perfusion (ChBP) to inhibit scleral hypoxia cascade reaction in the manufacture of a composition for delaying and inhibiting myopia onset and progression and treating myopia-related diseases. Preferably, the substance is the ω-3 polyunsaturated fatty acid.

The present invention also provides use of a ω-3 polyunsaturated fatty acid in the manufacture of a composition or device for regulating scleral lipid metabolism.

The present invention also provides use of a ω-3 polyunsaturated fatty acid in the manufacture of a composition for delaying and inhibiting myopia and myopia-related diseases by regulating scleral lipid metabolism.

The present invention also provides an ophthalmic injection, an ophthalmic gel, an ophthalmic ointment, an ophthalmic spray or an eye drop, wherein the active ingredient is the ω-3 polyunsaturated fatty acid.

The present invention provides use of ω-3 in preparing a drug in treating myopia by regulating scleral lipid metabolism. Optionally, the ω-3 is fish oil or cod liver oil; preferably, the ω-3 is DHA or EPA alone, or a combination of DHA and EPA.

The present invention also relates to a method for treating myopia, comprising: regulating the scleral lipid metabolism, or applying a substance or a device capable of regulating the scleral lipid metabolism pathway.

The present invention also relates to a method for diagnosing myopia in an individual or predicting a risk of myopia in an individual, wherein the status of the scleral lipid metabolism in the individual is detected, and if lipid deposition occurs in this site, the presence of myopia or a risk of myopia is determined; alternatively, the expression level of carnitine palmitoyl transferase 2 in an eye is detected, if the expression level of carnitine palmitoyl transferase 2 is reduced, the presence of myopia or a risk of myopia is determined.

As used herein, the term "prevent and control" refers to "prevent", "inhibit", "treat" or "alleviate" a disease or disorder, these terms can be interchangeable in specific positions herein, all expressing the meaning of realization of drug efficacy. Among them, "treat" or "alleviate" refers to therapeutic treatment measures and prevention or control measures, aiming at preventing or alleviating (relieving) a target disease or disorder. For example, after receiving the method of the present invention or a therapeutic amount of ocular sclera regulator or pharmaceutical composition of the present invention, a subject exhibits an observable and/or measurable reduction and disappearance of one or more conditions and symptoms of an ocular disease, or alleviation of disease progression, indicating that the subject's ocular disease is successfully treated. It also should be understood that, various modes of treating or preventing medical conditions described herein are intended to indicate "significant", which includes a complete treatment or prevention and a less complete treatment or prevention, wherein a biologically or medically relevant outcome is achieved. In some embodiments, "treatment" does not need 100% of elimination or reversion of myopia or myopia symptoms. In some embodiments, in contrast to the levels observed in the absence of the composition or method of the present invention (for example, in a biologically matched control subject or specimen that is not exposed to the composition of the present invention or the compound of the method of the present invention), "treatment" of myopia or myopia-related symptoms according to the method of the present invention achieves, for example, at least about 5%, at least about 10% or at least about 20% of alleviation, inhibition, blockage, prevention and/or reversion. In some embodiments, as compared to myopia or myopia-related symptoms in the absence of the compound of the present invention, at least about 30%, at least about 40%, at least about 50%, at least about 60%, at least about 70%, at least about 80%, at least about 90% or more (about 100%) of myopia or myopia-related symptoms are treated.

"Prevention and control" of a disorder or a disease refers to a fact that a compound, a pharmaceutical composition, a preparation, a device or a method in a statistical sample reduces occurrence of the disorder or the disease of the treated sample relative to an untreated control sample, or delays the occurrence of one or more symptoms of the disorder or the disease, or alleviates the severity of one or more symptoms of the disorder or the disease relative to the untreated control sample.

The regulation described herein refers to restoring the scleral lipid metabolism to be normal, or partially or basically restoring the level of scleral lipid metabolism to be normal.

The ω-3 of the present invention is the ω-3 polyunsaturated fatty acid.

In one embodiment, the substance is not ω-3, preferably, the substance is an SREBP-1cinhibitor or a carnitine acyl transferase 2 agonist. In one embodiment, the substance is ω-3, optionally, fish oil or cod liver oil; preferably, ω-3 is DHA or EPA, or a combination of DHA and EPA.

The lipid (or lipids) of the present invention comprises esters composed of fatty acid(s) (mostly long chain monocarboxylic acids with more than four carbons) and alcohol(s) (including glycerol alcohol, nitroamino alcohol, higher monohydric alcohol and sterol), and their derivatives, comprising simple lipids, composite lipids(compound lipids) and derivatives thereof, including but not limited to DHA and/or EPA.

The EPA of the present invention can be orally administered at a dose of 1 ng-30 g, preferably 1 µg-500 mg, or 10 mg-650 mg, for example, 10 mg, 20 mg, 30 mg, 40 mg, 50 mg, 60 mg,70 mg, 80 mg, 90 mg, 100 mg, 110 mg, or 120 mg, etc.

The DHA of the present invention can be orally administered at a dose of 1 ng-30 g, preferably 1 µg-500 mg, or 50 mg-1200 mg, for example, 50 mg, 100 mg, 150 mg, 200 mg, 250 mg, 300 mg, 350 mg, 400 mg, 450 mg, 500 mg, 550 mg or 650 mg, etc.

The content of EPA in the eye drop, the eye spray or the eye injection of the present invention is in a range of 1 ng-30 mg/100 µL, preferably 1 ng-10 µg/100 µL, for example, 1 µg/100 µL, 2 µg/100 µL, 3 µg/100 µL, 4 µg/100 µL, 5 µg/100 µL, 6 µg/100 µL, 7 µg/100 µL, 8 µg/100 µL, 9 µg/100 µL or 10 µg/100 µL, etc.

The content of DHA in the eye drop, the eye spray or the eye injection of the present invention is in a range of 1 ng-30 mg/100 µL, preferably 1 ng-10 µg/100 µL, for example, 1 µg/100 µL, 2 µg/100 µL, 3 µg/100 µL, 4 µg/100 µL, 5 µg/100 µL, 6 µg/100 µL, 7 µg/100 µL, 8 µg/100 µL, 9 µg/100 µL or 10 µg/100 µL, etc.

It is predicted that the dose for treating human myopia is 5-1000 times that of the above dose.

A mass ratio of DHA to EPA in the composition of DHA and EPA of the present invention (DHA:EPA or EPA:DHA) is 2000:1-1:2000, preferably, 500:1-1:500, more preferably, 50:1-1:3, or 1:1, 1:2, 1:3, 1:4, 1:5, 5:1, 4:1, 3:1, or 2:1, etc.

In a specific embodiment, the mass ratio of EPA to DHA in the composition of EPA and DHA of the present invention is: EPA:DHA = 5:1 to EPA:DHA = 99:1; preferably, EPA accounts for: 50% < EPA < 100%; preferably, EPA:DHA = 5:1 to EPA:DHA = 19:1; more preferably, EPA:DHA = 5:1 to EPA:DHA = 9:1; further preferably, EPA:DHA = 9:1, or EPA:DHA=5:1.

In a specific embodiment, the mass ratio of EPA to DHA in the composition of EPA and DHA of the present invention is: DHA:EPA = 5:1 to DHA:EPA = 99:1; preferably, DHA:EPA = 5:1 to DHA:EPA = 19:1; more preferably, DHA:EPA = 9:1, or DHA:EPA = 5:1.

In a mixture form of DHA and EPA, EPA is a predominant component (i.e., EPA:DHA > 1:1).

In a mixture form of DHA and EPA, DHA is a predominant component (i.e., DHA:EPA > 1:1).

The "predominant component" or "predominant active ingredient" described herein means that the proportion of a substance in the composition or all the active ingredients is more than 50% but less than 100%, for example, 60%, 70%, 80%, 90%, or 99%. In one embodiment, the proportion of EPA in the composition of DHA and EPA is 50% < EPA < 100%.

The scleral lipid metabolism signal pathway intervention regulator, or the scleral lipid metabolism regulatingsubstance, or the scleral lipid metabolism pathway regulating substance can be a compound, a drug, food, a preparation, a composition (or a pharmaceutical composition), a mixture, a complex or a device.

The drug used herein can also be referred to as a compound, a preparation, a composition, a pharmaceutical composition, a mixture or a complex.

The myopia-related diseases (complications) include vitreous opacity, retinal hemorrhage and detachment caused by myopia, complications of high myopia such as floaters, glaucoma, posterior staphylomas, retinal detachment, retinal tear, amblyopia, choroidal neovascularization, macular hemorrhage, choroidal atrophy, macular degeneration or denaturation, visual field defect, progressive or sudden decline of vision (especially near vision), eye soreness and/or pain, and night blindness.

In one embodiment, the individual or the subject is animal (such as a mammal) or human.

The myopia used herein can be refractive myopia and/or axial myopia. Preferably, the myopia is early myopia, and in one embodiment, the myopia is early myopia in adults; in one embodiment, the myopia is early myopia in children or adolescents.

The myopic individuals or individuals with a tendency to myopia refer to children and/or adolescents, preferably people aged 3-26 years, more preferably people aged 6-18 years; or refer to minors, preferably people whose eyes (eyeballs) are still in the stage of growth and development; or refer to school-aged children.

The drug or the composition of the present invention is prepared into an ophthalmic preparation, preferably, the ophthalmic preparation further comprises a pharmaceutically acceptable carrier, preferably the carrier is an ophthalmologically acceptable carrier. In one embodiment, the active substance in the composition is a derivative of the ω-3 polyunsaturated fatty acid. Preferably, the derivative of the ω-3 polyunsaturated fatty acid comprises a ω-3 polyunsaturated fatty acid ester.

Preferably, the drug is topically administered, especially is subjected to ocular administration or peribulbar injection. More preferably, the drug is administrated by eye drops. It is understood by those skilled in the art that the morphology and adaptability of the eyeball of an animal (e.g., mouse and guinea pig) are different from those of a human, because the former is protruding without depression. Therefore, the animal is commonly subjected to conjunctival injection or peribulbar injection to simulate the eye drop administration to a human.

The pharmaceutical dosage form of the present invention can be a liquid, a capsule, a granule, a powder, a tablet, an ointment, an emulsion, a suspension, etc. Further, the liquid dosage form is an injection (e.g., a peribulbar or intravitreal injection), an oral agent or an eye drop.

Optionally, the drug also contains a pharmaceutically acceptable adjuvant. Further, the pharmaceutically acceptable adjuvant are selected from an excipient, a disintegrant, an osmotic pressure regulator, a pH modifier, a viscosity modifier, a solubilizer, a stabilizer, a bacteriostatic agent and/or an antioxidant. Further, the pH modifier is selected from a borate buffer and a phosphate buffer. Further, the osmotic pressure regulator is sodium chloride, boric acid or borax. Further, the bacteriostatic agent is selected from phenylmercuric nitrate and phenylmercuric acetate. Further, the viscosity modifier is selected from methyl cellulose (MC), polyethylene glycol (PEG), polyethylene (PVA), and povidone (PVP). Further, the antioxidant is vitamin E.

In some embodiments, the ω-3 polyunsaturated fatty acid is selected from a combination of one or more of eicosapentaenoic acid (EPA), docosapentaenoic acid (DPA), docosahexaenoic acid (DHA) and/or α-Lindenic acid (ALA), such as a combination of DHA and EPA.

The composition of the present invention can be food. As a food form, various forms of food (such as health products, health food, and dietary supplements) can be recited, for example, a liquid form such as juice, soft drink, beverage or tea; a solid form such as biscuit, tablet, granular powder, powder or capsule; and a semi-fluid form, such as paste, jellie, soup or condiment.

The composition of the present invention can be an eye drop, such as an aqueous eye drop or suspended eye drop (e.g., a suspension). The eye drop can be mixed with a component such as a pharmacologically active ingredient and physiologically active ingredient. Such the component can be, for example, a decongestant component, an ocular muscle regulator component, an anti-inflammatory agent component, an astringent component, an antihistamine component, an antiallergic agent component, a vitamin, an amino acid, an antibacterial agent component, carbohydrates, a polymer compound or its derivative, a cellulose or its derivative, a local anesthetic component, a glaucoma treatment component, a cataract treatment component, and the like.

The preparation of the present invention can be an oral product such as a health care product, food, a dietary supplement, a nutritional product and drink, or a cosmetic; wherein, the cosmetic can be a combination of one or more of a free solution, an oil-water mixture, a suspension (agent), a liniment, a lotion, a spray, a cream, a drop, an electuary, an ointment, a paste, a pill, a suppository, an emulsion and a patch.

The device of the present invention is an instrument, equipment, a consumable, a system, a medical device, a health care product or an eye appearance-altering product that can release drugs or has a drug delivery function or has a potential of delivering drugs, such as a corneal contact lens, eyeglasses, an intraocular lense, a suture, an OK mirror cleaning (maintenance) system, an eye patch, an eyesight improving patch, a cosmetic contact lenses, a microneedle, an eye spray system, an eye massager, an eye fumigator, an eye surface drug delivery device, an intraocular drug delivery device, a fundus drug delivery device, an implant pump and a wearable device; alternatively, the device can be various glasses, corneal contact lenses (OK mirror, etc.) and frame glasses that can delay the progression of myopia, as well as instruments, equipment, consumables, medical instruments or health care products that have a vision protecting function or a myopia treatment (correction) effect, such as eye patches, (myopia) acupoint massagers, eye relaxation equipment and myopia therapeutic equipment.

In the process of implementing the present invention, the systemic administration dosage form (e.g., an oral tablet) and the topical administration dosage form (e.g., an eye drop) are used simultaneously, or in combination, or alternately, or at intervals, or alone.

Any drug or the preparation involved in the method of the present invention can be administered in the same period, such as simultaneously or successively administered in a specific administration (treatment) process, administered on the same day, administered in the same week, administered in the same month and administered in the same year; or can be alternately administered at intervals, such as at an interval of 4 hours, or at an interval of 12 hours, or alternately administered every other day, alternately administered every other week, alternately administered every other month, alternately administered every other year, etc.

The preparation or the drug of the present invention can be used in combination with one or more drugs for preventing or treating myopia, for example, is formulated or designed in the form of continuous administration, simultaneous administration, successive administration, alternate administration, interval administration, or alone administration.

The preparation or the drug of the present invention can be used in combination with one or more other drugs. For example, the other drugs are drugs for preventing and controlling and/or treating myopia (M receptor blockers such as dibazole, polyunsaturated fatty acids, prazosin, salidroside and atropine, and 7-methyl xanthine, and nicotinic acid), vasodilators, smooth muscle relaxers, drugs for preventing vasospasm, drugs for regulating collagen metabolism, Piracetam, antiallergic drugs, liver-protecting drugs, or combinations thereof.

The drug or the pharmaceutical composition, the preparation or the device of the present invention also comprises other ophthalmic preparations or drugs, including but not limited to drugs for treating myopia, M receptor blockers (such as M3 receptor blockers or antagonists or inhibitors, atropine), dibazole, polyunsaturated fatty acids (such as DHA and EPA), salidroside, prazosin, homatropine, anisodamine (racemic), topicamide, 7-methyl xanthine, nicotinic acid, Piracetam, red a sage root extract, a safflower extract, fish oil, a bear bile extract, vitamins, adenosine triphosphate (ATP), and therapeutic components and adjuvants for ophthalmic diseases.

The drug or the pharmaceutical composition or the preparation of the present invention can be an injection, a tablet, a lyophilized powder injection, a capsule, an effervescent tablet, a chewable tablet, a buccal tablet, a granule, an ointment, a syrup, an oral liquid, an aerosol, a nasal drop, an external preparation, an oral preparation, etc; preferably, the drug or the pharmaceutical composition or the preparation of the present invention can be an ophthalmic dosage form, including but not limited to an eye drop (an eye solution), an eye ointment, an eye spray, an implant, an ophthalmic gel, an eye patch, an ophthalmic microsphere, an ophthalmic sustained-release preparation, a periocular injection, an intraocular injection; the drug or the pharmaceutical composition or the preparation of the present invention can also be a free solution, an oil-water mixture, a suspension (agent), a liniment, a lotion, a cream, a drop, an electuary, a spray, an ointment, a patch, a paste, a pill, a suppository, an emulsion, and a formulated composition containing cellulose (such as methylcellulose), a dendrimer, nanomaterial, sustained-release material, liposome or a combination thereof.

The preparation or the drug of the present invention can be adopted in combination with a device and/or a surgery, such as an orthokeratology, a refractive correction surgery, a myopia corneal laser surgery, and a crysalline lens surgery.

The preparation or the drug of the present invention can be administered systemically (e.g., orally, or intravenously), and/or topically (eye drop, intravitreal injection, skin ointment/emulsion eye application or eye ointment application), and/or parenterally (such as mucosal administration and transdermal administration).

The myopia involved in the present invention can be refractive myopia or axial myopia; congenital myopia (myopia at birth or before school age), early-onset myopia (under 14 years old), delayed myopia (16-18 years old), late-onset myopia (after adulthood); low myopia (mild myopia), moderate myopia, high myopia (severe myopia); pseudomyopia, true myopia; childhood and/or adolescent myopia (preferably 3-26 years old, more preferably 6-18 years old), myopia in minors, myopia in adults, myopia in the elderly; simple myopia, pathological myopia; axial simple myopia, simple axial myopia; axial myopia in children and/or adolescents (preferably 3-26 years old, more preferably 6-18 years old); axial myopia in school-aged and preschool-aged populations; primary myopia, secondary myopia; primary myopia in children and/or adolescents(preferably 3-26 years old, more preferably 6-18 years old); or progressive myopia in children and/or adolescents (preferably 3-26 years old, more preferably 6-18 years old).

Individuals involved in myopia inhibition in the present invention can be children and/or adolescents, preferably people aged 3-26 years, more preferably people aged 6-18 years; or refer to minors, preferably people whose eyes (eyeballs) are still in the stage of growth and development.

The present invention has the beneficial effects that by regulating the scleral lipid metabolism, the occurrence and development of myopia are effectively delayed and inhibited, the ocular axis elongation is inhibited and the vitreous chamber depth is increased; furthermore, by local administration (such as by periocular injection to an animal), the effect of a milligram-level oral administration can be achieved when the content of the ω-3 polyunsaturated fatty acid is in a microgram level, which well avoids the health risks brought by systemic administration (cod liver oil). At the same time, the present invention provides an optimal therapeutic scheme (such as eye drops containing DHA and EPA in a specific ratio) and avoids adverse reactions such as allergies.

### BRIEF DESCRIPTION OF DRAWINGS

FIG. 1 is a sclera transcriptome sequencing map of mice with form-deprivation myopia, wherein, "Treat" indicates a form-deprivation treated eye; "Fellow" indicates a fellow eye of the form-deprived eye; "Control" indicates an untreated control eye.
FIG. 2 is an electron microscopy of sclera in mice with form-deprivation myopia.
FIG. 3 is a diagram showing refraction difference between an experimental eye and a fellow eye in intragastric administration group. Refraction: diopter; FD + vehicle: form deprivation + edible olive oil treatment group (solvent group); FD + ω-3: form deprivation + (DHA 300 mg, EPA 60 mg) treatment group (administration group).
FIG. 4 is diagram showing a vitreous chamber depth difference between an experimental eye and a fellow eye in intragastric administration group, VCD: vitreous chamber depth; FD + vehicle: form deprivation + edible olive oil treatment group (solvent group); FD + ω-3: form deprivation + ω-3 polyunsaturated fatty acid (DHA 300 mg, EPA 60 mg) treatment group (drug administration group).
FIG. 5 is a diagram showing an axis length difference between an experimental eye and a fellow eye in the intragastric administration group, AL: axis length; FD + vehicle: form deprivation + edible olive oil treatment group (solvent group); FD + ω-3: form deprivation + ω-3 polyunsaturated fatty acid (DHA 300 mg, EPA 60 mg) treatment group (drug administration group).

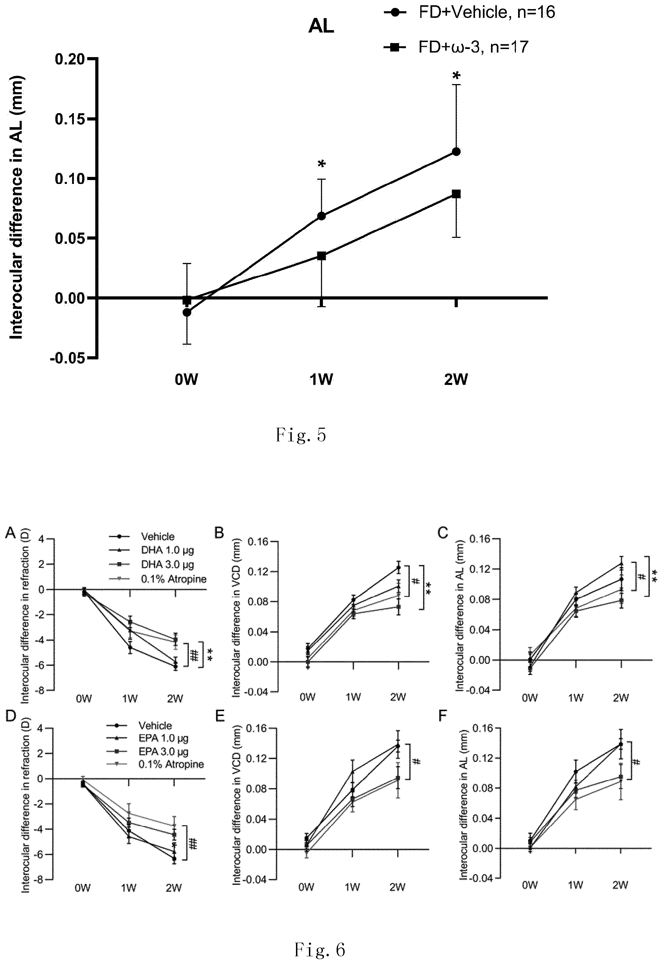

FIG. 6 is a diagram showing vitreous chamber depth difference, vitreous chamber depth difference and axis length difference between an experimental eye and a fellow eye in peribulbar injection group, wherein, refraction: diopter; VCD: vitreous chamber depth; AL: axis length; FIG. 6A shows binocular refraction difference among low-dose and high-dose DHA groups, Vehicle group and 0.1% atropine group (positive control group) by injection; FIG. 6B shows binocular difference in VCD among low-dose and high-dose DHA groups, Vehicle group and 0.1% atropine group by injection; FIG. 6C shows binocular difference in AL among low-dose and high-dose DHA groups, Vehicle group and 0.1% atropine group by injection; FIG. 6D shows binocular difference in refraction among low-dose and high-dose EPA groups, Vehicle group and 0.1% atropine group (positive control group) by injection; FIG. 6E shows binocular difference in VCD among low-dose and high-dose EPA groups, Vehicle group and 0.1% atropine group by injection; FIG. 6F shows binocular difference in AL among low-dose and high-dose EPA injection groups, Vehicle group and 0.1% atropine group by injection.

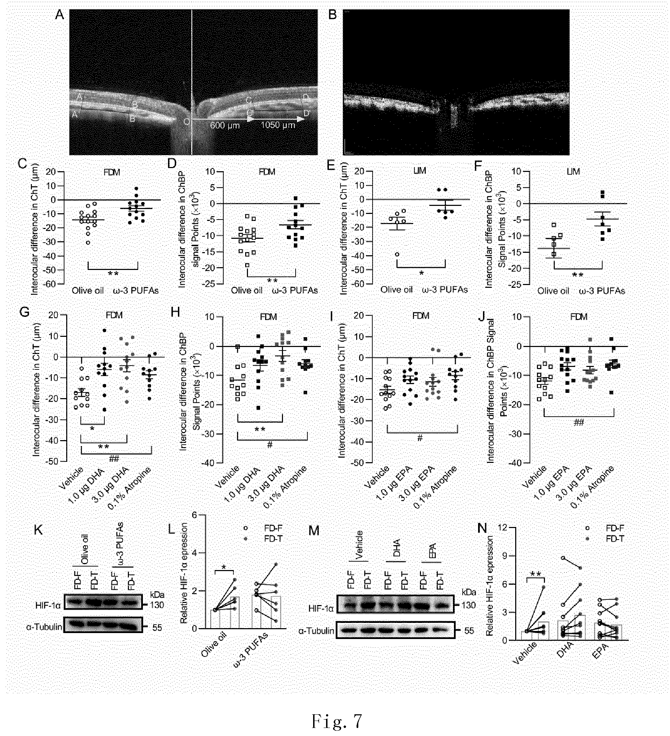

FIG. 7 is a diagram showing difference in ChT and ChBP between an experimental eye and a fellow eye after different treatments as well as the detection results of HIF-1α protein expression, wherein, FIG. 7A is a schematic diagram of OCT assay, where lines AB and CD show the inner surface of the choroid and O represents the optic disc; AA' and BB' indicate nasal/inferior choroidal thickness; and CC' and DD' indicate temporal/superior choroidal thickness. FIG. 7B is an OCTA image, where the bright part indicates the perfusion signal point; FIG. 7C shows ChT difference between the experimental eye and the fellow eye after FD treatment plus 2 weeks of feeding olive oil and ω-3 polyunsaturated fatty acids; FIG. 7D shows ChBP difference between the experimental eye and the fellow eye after FD treatment plus 2 weeks of feeding olive oil and ω-3 polyunsaturated fatty acids; FIG. 7E shows ChT difference between the experimental eye and the fellow eye after LIM treatment plus 2 weeks of feeding olive oil and ω-3 polyunsaturated fatty acids; FIG. 7F shows ChBP difference between the experimental eye and the fellow eye after LIM treatment plus 2 weeks of feeding olive oil and ω-3 polyunsaturated fatty acids; FIG. 7G shows interocular ChT difference after 2 weeks of injection on FD eyes with Vehicle, 1.0 µg DHA, 3.0 µg DHA, or 0.1% atropine, respectively; FIG. 7H shows interocular ChBP difference after 2 weeks of injection on FD eyes with Vehicle, 1.0 µg DHA, 3.0 µg DHA, or 0.1% atropine; FIG. 7I shows difference in ChT between the experimental eye and the fellow eye after 2 weeks of injection on FD eyes with Vehicle, 1.0 µg EPA, 3.0 µg EPA or 0.1% atropine; FIG. 7J shows difference in ChBP between the experimental eye and the fellow eye after 2 weeks of injection on FD eyes with Vehicle, 1.0 µg EPA, 3.0 µg EPA or 0.1% atropine; FIG. 7K and FIG. 7L show the results of western blotting on the interocular HIF-1α protein and related protein expression after FD treatment plus 2 weeks of feeding olive oil and ω-3 polyunsaturated fatty acids, where FD-F indicates the fellow eye, and FD-T indicates the FD experimental eye; FIG. 7M and FIG. 7N show the results of western blotting on the interocular HIF-1α protein expression after 2 weeks of injection on FD eyes with Vehicle, DHA or EPA, where FD-F indicates the fellow eye, and FD-T indicates the FD experimental eye.
FIG. 8 shows a clinical trial of the effect of oral administration of cod liver oil on ChBP before and after near-distance work in humans, wherein, FIG. 8A indicates an operational flow of near-distance work; FIG. 8B indicates changes in ChT; FIG. 8C indicates changes in a stomal zone; FIG. 8D indicates changes in a vascular luminal zone; FIG. 8E indicates changes in a choroidal vascularity index; FIG. 8F indicates changes in the area of a choroidal non-perfused zone.
FIG. 9 shows a safety study on the effects of intragastric administration of ω-3polyunsaturated fatty acids on anterior chamber depth (ACD), crystal thickness (LT) and body weight (Weight).
FIG. 10 shows a safety study on the effects of peribulbar injection of DHA and EPA on anterior chamber depth (ACD), lens thickness (LT), and radius of corneal curvature (RCC).
FIG. 11 shows an allergy study: A: form deprivation + fish oil group; B: form deprivation + drug group (DHA alone or DHA + EPA).
FIG. 12 shows a study on the effectiveness of an optimal treatment scheme, wherein the effects of peribulbar injection of polyunsaturated fatty acids with different proportional formulations on refraction (FIG. A), vitreous chamber depth (FIG. B) and eye axis length (FIG. C) are studied.
FIG.13 shows a study on the safety of an optimal treatment scheme, wherein the effects of peribulbar injection of polyunsaturated fatty acids with different proportional formulations on refraction (FIG. A), crystal thickness (FIG. B) and radius of corneal curvature (FIG. C) are studied.

In the above figures, "difference" refers to the difference in refraction or eye axial parameters between the experimental eye and the fellow eye; variance analysis based on repeated measurement data are used for comparison between solvent and drug administration groups: "^{∗}" indicates P<0.05; "^{∗ ∗}" indicates P<0.01; "^{∗ ∗ ∗}" indicates P<0.001. ^{∗} denotes a statistical difference between ω-3 polyunsaturated fatty acids/DHA/EPA treatment and the solvent control; # denotes a statistical difference between atropine treatment and solvent control.

### DETAILED DESCRIPTION OF THE INVENTION

### Example 1 Close connection between abnormal scleral metabolism and myopia

The test animals were C57BL6 mice aged 3 weeks, and subjected to monocular form deprivation (FD) by an eyeshade method, one group of animals were anesthetized and killed after experiment for 2 days, and the binocular scleras were taken for transcriptome sequencing, while another group of animals were taken for electron microscopic observation after experiment for 2 weeks.

As seen from FIG. 1, after form deprivation for 2 days, a scleral lipid metabolism signaling pathway in myopic eyes of mice show significant changes, as compared to the fellow eyes, and a key lipid metabolism enzyme at this site, carnitine palmitoyl transferase 2 (Cpt2), is significantly reduced, indicating the downregulation of the scleral lipid metabolism pathway in myopic eyes.

As seen from FIG. 2, after form deprivation for 2 weeks, scleral lipid deposition increases in myopic eyes of mice as compared to fellow eyes, indicating abnormal scleral lipid metabolism in myopic eyes.

### Example 2 A scleral metabolism regulating substance is capable of inhibiting myopia

The test animals were British tricolored short-haired guinea pigs aged 3 weeks. The guinea pigs were subjected to monocular form deprivation (FD) by a mask method, and allowed to an intragastric administration of ω-3 polyunsaturated fatty acid. The animals were randomly divided into 2 groups: FD + solvent control group (FD + vehicle) (a solvent here was edible olive oil); and FD + drug group (FD + ω-3 (DHA 300 mg, EPA 60 mg)). Intragastric administration was performed at 9 a.m., continuing for 2 weeks. Before test, and administration for 1 week and 2 weeks, respectively, refraction was measured by an eccentric infrared refractometer (EIR), ocular axis parameters such as vitreous chamber depth and axial length were measured by A-scan (11 MHz), and scleral lipid metabolism was analyzed by gas chromatography-mass spectrometry (GC-MS).

Comparing the measured parameters before and after the experiment, it was found that FD eyes, degrees of refractive myopia, vitreous cavity elongation and eye axis elongation in administration group are smaller than those in FD control group and solvent administration group, and were statistically significant as compared to solvent control group, moreover, the scleral lipid metabolism level was partly restored or basically restored to be normal. Therefore, feeding ω-3 polyunsaturated fatty acids can inhibit the formation of FD myopia in guinea pigs or slow down the development of FD myopia in guinea pigs.

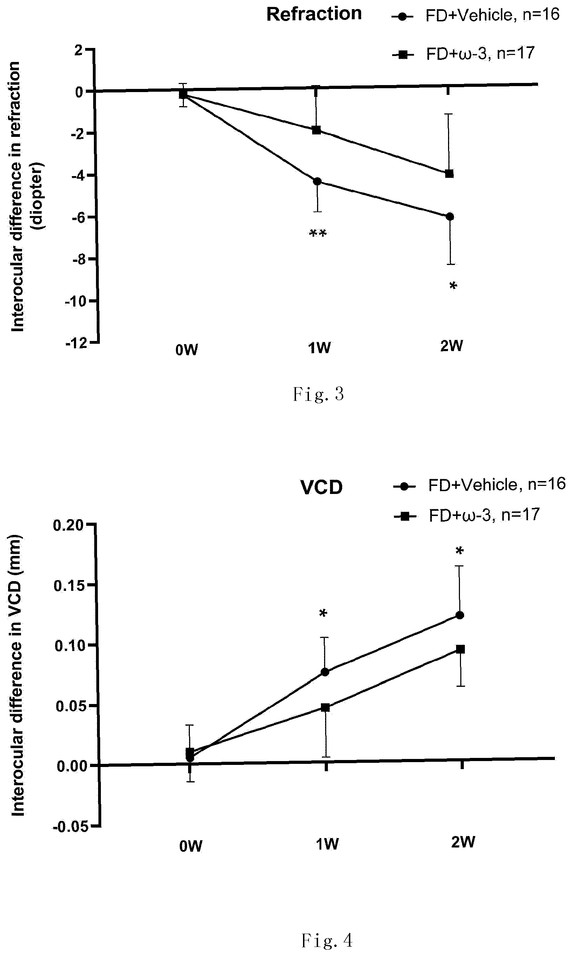

As shown in FIG. 3, after 2 weeks of experiment, the degree of refractive myopia in administration group is less than that in solvent group, and there is a temporal effect, indicating that the ω-3 polyunsaturated fatty acid can inhibit the progression of FD myopia.

As shown in FIG. 4, after 2 weeks of experiment, the vitreous cavity elongation in administration group is significantly less that in solvent group, and there is a temporal effect, indicating that the ω-3 polyunsaturated fatty acid can inhibit the vitreous cavity elongation in FD treated eyes.

As shown in FIG. 5, after 2 weeks of experiment, the ocular axis elongation in administration group is significantly less that in solvent group, and there is a temporal effect, indicating that the ω-3 polyunsaturated fatty acid can inhibit the ocular axis elongation in FD treated eyes.

The above experiments prove that the ω-3 polyunsaturated fatty acid can significantly play a role in delaying negative refraction and eye axis elongation.

### Example 3 A scleral metabolism regulating substance can inhibit negative refraction and eye axis elongation of myopic eyes

The test animals were British tricolored short-haired guinea pigs aged 3 weeks. The animals were subjected to monocular form deprivation (FD) by a mask method and were randomly divided into 6 groups, which were treated by peribulbar injection with the following different substances: (1) ethanol solvent group (Vehicle); (2) low-dose DHA group (1.0 µg); (3) high-dose DHA group (3.0 µg); (4) low-dose EPA group (1.0 µg); (5) high-dose EPA group (3.0 µg); and (6) 0.1% atropine group.

The measurement methods of refraction, vitreous chamber depth and eye axis length were the same as those in Example 2.

As seen from FIG. 6, form deprivation successfully induces myopia in guinea pigs after two weeks of injection, and the myopia progression of the treated guinea pigs in high-dose DHA group is reduced by 35.3% compared to that in Vehicle group (FIG. 6A), accompanied by significant reduction in both VCD and AL elongation, and the effect is more apparent with the extension of time; likewise, similar results are obtained in high-dose EPA group.

In summary, it can be seen from the above experiments that peribulbar injection with high-dose ω-3 polyunsaturated fatty acids (3 µg/day) can play roles in inhibiting the negative refraction and eye axis elongation. Topical administration of ω-3 polyunsaturated fatty acids can delay myopia progression.

### Example 4 ω-3 polyunsaturated fatty acids inhibit myopia by inhibiting ChBP reduction and sclera hypoxia cascade reaction

ChT and ChBP of guinea pigs were detected by optical coherence tomography (OCT) and optical coherence tomography angiography (OCTA), and the HIF-1α protein expression levels in different treatments were detected by western blotting.

The test animals were British tricolored short-haired guinea pigs aged 3 weeks, and were subjected to monocular form deprivation (FD) by a mask method or subjected to monocular lens induction (LI), and divided into 3 groups for test: (1) the FD treated guinea pigs were fed with ω-3 polyunsaturated fatty acids and olive oil control, and the interocular (between the experimental eye and the fellow eye, similar hereinafter) differences in ChT and ChBP were compared, respectively; (2) the LI treated guinea pigs were fed with ω-3 polyunsaturated fatty acids and olive oil control, and the interocular (between the experimental eye and the fellow eye, similar hereinafter) differences in ChT and ChBP were compared, respectively; (3) the FD treated guinea pigs were subjected to peribulbar injection, and randomly divided into 6 groups: (a) ethanol solvent group (Vehicle); (b) low-dose DHA group (1.0 µg); (c) high-dose DHA group (3.0 µg); (d) low-dose EPA group (1.0 µg); (e) high-dose EPA group (3.0 µg); (f) 0.1% atropine group, and the interocular differences in ChT and ChBP were compared, respectively.

As seen from FIG. 7C and FIG. 7D, the ChT of the FD treated guinea pigs fed with olive oil treatment group is significantly reduced accompanied by reduction in ChBP, however, compared to olive oil treatment group, feeding with ω-3 polyunsaturated fatty acids can significantly inhibit reduction in both ChT and ChBP. As seen from FIG. 7E and FIG. 7F, the LI treated guinea pigs also show a similar inhibitory effect.

As seen from FIG. 7G and FIG. 7H, treatment groups injected with DHA, regardless of high-dose group and low-dose group, significantly inhibit the reduction in both ChT and ChBP, as compared to Vehicle group. As shown in FIG. 7I and FIG. 7J, treatment groups injected with EPA have no significant effects, but according to data in the figures, both high-dose group and low-dose groupshow a certain inhibitory effect.

It is reported that increasing ChT and ChBP can inhibit the development of myopia (X. Zhou et al., Increased Choroidal Blood Perfusion Can Inhibit Form Deprivation Myopia in Guinea Pigs. Invest. Ophthalmol. Vis. Sci. 61, 25 (2020)). Combined with the above experimental results, it can be seen that ω-3 polyunsaturated fatty acids can delay and inhibit the development of myopia by inhibiting reduction in ChT and ChBP.

Scleral hypoxia and upregulation of HIF-1α expression promote transdifferentiation of myofibroblasts and remodeling of extracellular matrix (ECM), leading to the occurrenceand development of myopia (H. Wu et al., Scleral hypoxia is a target for myopia control. Proc. Natl. Acad. Sci. U. S. A. 115, E7091-E7100 (2018); F. Zhao et al., Scleral HIF-1alpha is a prominent regulatory candidate for genetic and environmental interactions in human myopia pathogenesis. EBioMedicine 57, 102878 (2020)). As shown from FIG. 7K and FIG. 7L, in guinea pigs fed with olive oil, the HIF-1α protein level in the sclera of the experimental eyes (FD-T) is higher than that in the fellow eyes (FD-F), while the increase of HIF-1α protein level in the sclera of the experimental eyes in guinea pigs fed with ω-3 polyunsaturated fatty acids is inhibited. Similarly, according to FIG. 7M and FIG. 7L, in peribulbar injection treatment group, the increase of the HIF-1α protein level in guinea pigs injected with DHA or EPA is inhibited. Combined with the above experiments, it can be seen that ω-3 polyunsaturated fatty acids can inhibit the scleral hypoxic cascade reaction during the myopia development, and then inhibits the development of myopia.

### Example 5 ω-3 polyunsaturated fatty acid can improve reduction in ChBP caused by human's near-distance work

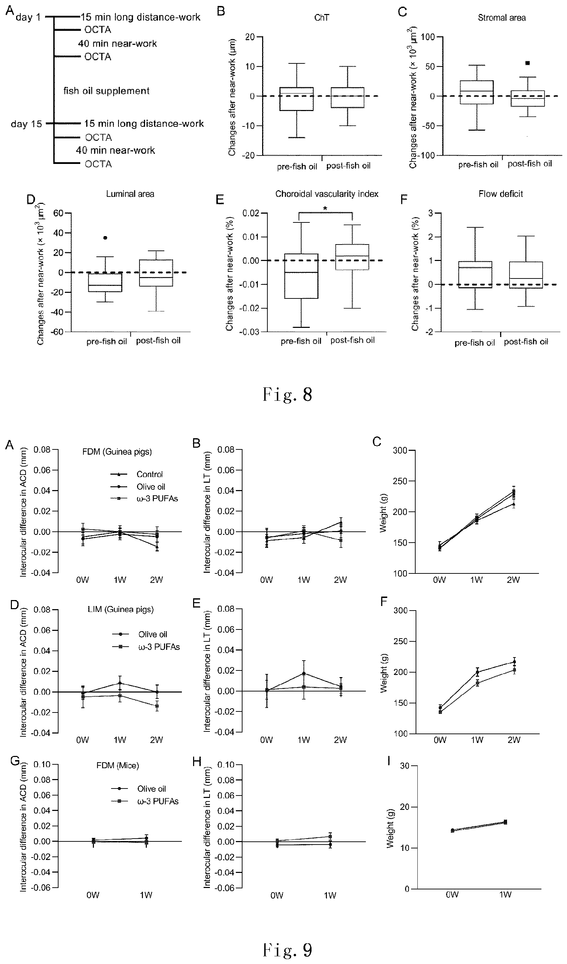

To verify the effect of ω-3 polyunsaturated fatty acid on human myopia, a clinical trial was implemented. The clinical trial was approved by the Ethics Committee of the Eye Hospital of Wenzhou Medical University, and the participants were first-year college students in Wenzhou Medical University. Experimental procedure: first, participants were allowed to watch TV at 3 meter distance for 15 minutes, and then subjected to OCTA measurements on choroidal thickness and the areas of the choroidal stomal area, vascular area, and non-perfused area, after which the participants were allowed to read with an electronic display at 33 cm distance for 40 minutes, and then subjected to the above OCTA measurements; after that, the participants were asked to take a fish oil capsule containing 600 mg DHA and 120 mg EPA daily, continuing for 14 days; on day 15, the choroidal data after reading 40 minutes were detected, respectively (FIG. 8A).

As can be seen from analysis, near-distance reading has no significant effect on changes in ChT and the stomal area (FIG. 8B and FIG. 8C), but shows a significant decrease in the alteration of the vascular luminal area and the alteration of the choroidal vascularity index (FIG. 8D and FIG. 8E), moreover, the near-distance reading has a significant effect on the area of the choroidal nonperfused zone, implying an increase in an area without a blood flow signal (FIG. 8F), all of which indicate that near-distance reading can reduce ChBP. Supplementation of fish oil can significantly delay the decrease in choroidal vascular index (FIG. 8E) and improve the reduction in the vascular luminal zone (FIG. 8D) and the increase in the area of nonperfused zone (FIG. 8F) to some extents. The above results indicate that ω-3 polyunsaturated fatty acids can inhibit the reduction in ChBP caused by human's near-distance work.

### Example 6 High-dose DHA is significantly superior to EPA, resulting in unpredicted efficacy

Peribulbar injection of either DHA or EPA can inhibite the development of myopia in guinea pigs. At the same dose, DHA shows a stronger inhibitory effect than EPA.

FD induces significant myopia in both solvent- and DHA-treated eyes of guinea pigs, including low-dose (1 µg/day) and high-dose DHA (3 µg/day) treatments (FIG. 6A). However, after two weeks of treatment, myopia progression in high-dose DHA treatment group is 35.3% less than that in solvent control group (PP < 0.01, FIG. 6A). This inhibitory effect is accompanied by significant reduction in VCD and AL elongation (FIG. 6B and FIG. 6C). Administration of atropine is a widely accepted drug treatment, which can inhibit the progression of myopia in human. Atropine (0.1%) treatment, as a positive control, has a reduced myopia rate of 35.6%, as compared with drug treatment group (PP < 0.01, FIG. 6A). Thus, it is proved that the inhibitory effect of atropine is similar to that of high-dose DHA. That is, these results suggest that peribulbar injection of guinea pigs with DHA can inhibit the development of FDM.

The trend for peribulbar injection of EPA is similar to that of DHA, but with a relatively weak inhibitory effect. After two weeks of treatment, administration of high-dose EPA (3.0 µg/day) shows 29.6% inhibition on the development of FD induced myopia, which has no statistically difference compared to negative control group, and its efficacy is lower than that of 0.1% atropine (FIG. 6D). Two doses of EPA have no significant effects on VCD or AL elongation (FIG. 6E and FIG. 6F).

### Example 7 Good saftey

All grouped animals in Example 2 were subjected to safety test. The results show that there are no significant differences in ACD, LT and body weight between eye group fed with ω-3 polyunsaturated fatty acids and Vehicle control group (FIG.9), indicating that ω-3 (DHA 300 mg, EPA 60 mg) is not potentially toxic to other organs, and has a good safety.

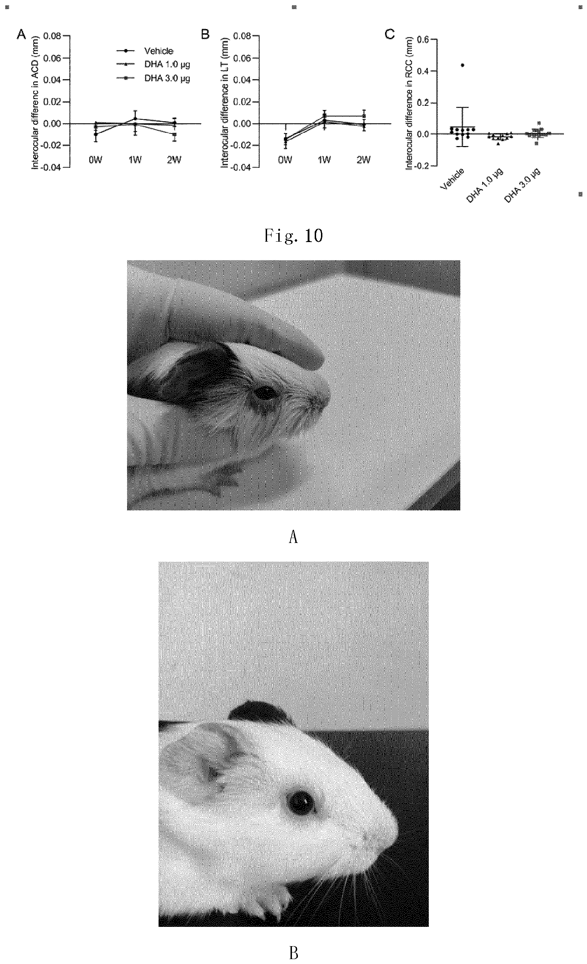

Similarly, there are no significant ocular differences in ACD, LT and body weight between eyes treated with DHA alone in Example 6 and Vehicle control group (FIG. 10), indicating that even a high dose of DHA (3 µg/day) is not potentially toxic to eyeballs, and has a good safety.

### Example 8 Avoidance of allergies

Animals were randomly divided into three groups: FD + cod liver oil (commercially available ω-3 polyunsaturated fatty acids) group; FD + high-purity drug group 1 (FD + DHA 3.0 µg); and FD + high-purity drug group 2 (FD + "DHA 3.0 µg + EPA 3.0 µg"), and both high-purity drug groups were at a high dose.

After applying a topical anesthetic (one drop of 0.5% propazocaine hydrochloride, Alcon Laboratories, Inc., Puurs, Belgium), 100 µL of drug was daily administered at the surrounding area of FD eyeballs at 9:00 a.m., continuing for 2 weeks. All injections were completed within 10 seconds under dark red light to minimize any possible impact of red light on induced myopic recovery.

It is found that periocular swelling occurs after 1-3 days of injection on animals in FD + cod liver oil group (FIG. 11A), and conjunctivitis occurs after 1 week; whereas animals in high-purity groups (DHA alone or DHA + EPA) have no adverse reactions such as allergies (FIG. 11B).

### Example 9 Optimal treatment scheme

The optimal treatment protocol or scheme applying ω-3 by means by of feeding and peribulbar injection were investigated, respectively, and some critical findings were shown, especially when the eye was subjected to topical administration, unexpected results were obtained by using DHA alone or a composition of DHA and EPA. The basic experimental procedures were as described above.

Regardless of the economy, "the composition of DHA and EPA containing EPA as a predominant component" is significantly more effective than ω-3 polyunsaturated fatty acids in other forms, if only considering the most critical therapeutic effects. That is unexpected. Based on previous experimental results and experience (e.g., Example 6), the myopic inhibition effect of DHA alone was stronger than that of EPA alone. Therefore, for a mixture of DHA and EPA, it should be reasonable to conclude that, the higher the proportion of DHA (e.g., 99% or more), the better the effect.

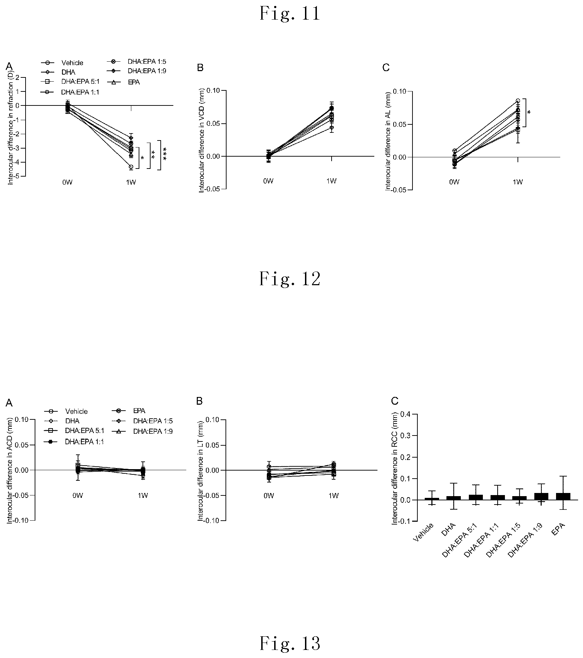

However, the inventors surprisingly find that in young guinea pig models with FD myopia, the mypia inhibition effect of DHA alone is still superior to that of EPA alone, and the treatment efficacy of DHA alone is reduced after mixing of EPA in DHA (the total mass of ocular topical administration is 3 µg in each case). However, as the content of EPA increases to become the predominant component (i.e., DHA:EPA < 1:1), the efficacy of the mixture of DHA and EPA (the total mass of ocular topical administration is 3 µg in each case) is even better than that of DHA alone, EPA alone, or a mixture of DHA and EPA containing DHA as the main component (i.e., DHA:EPA > 1:1, for example, DHA: EPA =1:5, or DHA:EPA = 1:9) and is statistically significant (Table1 and FIG. 12). It can be seen that the mixture of DHA and EPA that uses EPA as the predominant component or the predominant active ingredient has the optimal inhibition effect of myopia and myopia-related symptoms. Overall, all the treatment protocols in this example are very effective in inhibiting the negative refraction, eye axis elongation, and vitreous cavity depth increase.

Meanwhile, there are no significant interocular differences or statistical differences in ACD, LT and RCC between eye groups treated with polyunsaturated fatty acids in different proportional formulations and Vehicle control group (FIG.13). It can be seen that ocular topical administration of various mixture forms of DHA and EPA (e.g., 3 µg/day) is not potentially toxic to eyes, and is very safety.

**Table 1**

| Sample size | | 12 | 21 | 12 | 12 | 12 | 11 | 25 |
|---|---|---|---|---|---|---|---|---|
| Refraction | | Veh | DHA | DHA:EPA 5:1 | DHA:EPA 1:1 | DHA:EPA 1:5 | DHA:EPA 1:9 | EPA |
| | 0W | 0.02 | -0.23 | -0.20 | -0.21 | -0.06 | 0.17 | -0.40 |
| | 1W | -4.33 | -2.95 | -3.06 | -3.19 | -2.67 | -2.27 | -3.42 |
| Myopia inhibition rate | | | 31.8% | 29.4% | 26.3% | 38.3% | 47.5% | 21.0% |
| Vitreous chamber depth | 0W | 0.00 | 0.00 | 0.00 | 0.00 | 0.00 | 0.00 | 0.00 |
| | 1W | 0.07 | 0.06 | 0.06 | 0.07 | 0.06 | 0.05 | 0.07 |
| Eye axis length | 0W | 0.01 | -0.01 | -0.01 | 0.00 | -0.01 | -0.01 | 0.01 |
| | 1W | 0.08 | 0.06 | 0.04 | 0.07 | 0.06 | 0.04 | 0.07 |

As known to those skilled in the art, since human eyes are much larger than animal (such as guinea pigs) eyes and specific ocular tissue structures are different, it is predicted that the dose for optimal myopic treatment in human is 5-1000 times the dose specifically administered in the above examples, which can be achieved by increasing the single dose and/or increasing the frequency of daily dose clinically.

Therefore, the above description of the specific embodiments of the present invention discloses the technical details of the present invention in detail, exemplarily gives the technical thinking of the present invention, and is intended to satisfy the authorization provision of the patent law, but should not be considered as limiting the scope of protection of the present invention. Various changes or deformations can be made by researchers in the light of the present application in combination with the knowledge and technology at that time, and shall fall within the protection of the appended claims without departing from the core ideas and spirit of the present application.

## Claims

1. Use of a ω-3 polyunsaturated fatty acid, an SREBP-1c inhibitor and/or a carnitine palmitoyltransferase 2 agonist in the manufacture of a drug, a preparation or a device for regulating eye scleral lipid metabolism in an individual.

2. The use according to claim 1, wherein the device is an instrument, equipment, a consumable, system, a medical device, a health care product or an eye appearance-altering product that can release a drug or has a drug delivery function or has a potential of delivering drugs, such as a corneal contact lens, eyeglasses, an intraocular lens, a suture, an OK mirror cleaning (maintenance) system, an eye patch, an eyesight improving patch, a cosmetic contact lens, a microneedle, an eye spray system, an eye massager, an eye fumigator, an eye surface drug delivery device, an intraocular drug delivery device, a fundus drug delivery device, an implant pump and a wearable device; or
the device is various glasses, corneal contact lenses (OK mirror, etc.) and frame glasses that can delay the progression of myopia, as well as instruments, equipment, consumables, medical instruments or health care products that have a vision protecting function or a myopia treatment (correction) effect, such as eye patches, (myopia) acupoint massagers, eye relaxation equipment and myopia therapeutic equipment.

3. The use according to claim 1, wherein the preparation is an oral product such as a health care product, food, a dietary supplement, a nutritional product and drink, or a cosmetic; wherein, the cosmetic is a combination of one or more of a free solution, an oil-water mixture, a suspension (agent), a liniment, a lotion, a spray, a cream, a drop, an electuary, an ointment, a paste, a pill, a suppository, an emulsion and a patch.

4. The use according to claim 1, wherein the drug or the preparation is a liquid (such as an injection), a tablet, a lyophilized powder injection, a capsule, an effervescent tablet, a chewable tablet, a buccal tablet, a granule, an ointment, a syrup, an oral liquid, an aerosol, a nasal drop, an external preparation, an oral preparation, etc; preferably, the drug or the preparation is an ophthalmic dosage form, including but not limited to an eye drop (an eye solution), an eye ointment, an eye spray, an implant, an ophthalmic gel, an eye patch, an ophthalmic microsphere, an ophthalmic sustained-release preparation, a periocular injection, an intraocular injection; further, the drug or the preparation is a free solution, an oil-water mixture, a suspension (agent), a liniment, a lotion, a cream, a drop, an electuary, a spray, an ointment, a patch, a paste, a pill, a suppository, a powder, an emulsion, and a formulated composition containing cellulose (such as methylcellulose), a dendrimer, a nanomaterial, a sustained-release material, a liposome or a combination thereof.

5. The use according to one of claims 1-4, wherein the drug or the preparation is administered systemically (e.g., orally, and intravenously), and/or topically (by dropping into eyes, intravitreal injection, skin ointment/emulsion periocular application or eye ointment application), and/or parenterally (such as mucosal administration and transdermal administration).

6. The use according to claim 5, wherein the systemic administration dosage form (e.g., an oral capsule) and the topical administration dosage form (e.g., an eye drop) are used simultaneously, or in combination, or alternately, or at intervals, or alone.

7. The use according to one of claims 1-6, wherein the drug, the preparation or the device is administered in combination with other drugs, and the other drugs are drugs for preventing and controlling and/or treating myopia (M receptor blockers such as dibazole, polyunsaturated fatty acids, docosahexaenoic acid (DHA), fish oil, prazosin, salidroside and atropine, 7-methyl xanthine, and nicotinic acid), vasodilators, smooth muscle relaxers, drugs for preventing vasospasm, drugs for regulating collagen metabolism, Piracetam, antiallergic drugs, liver-protecting drugs, or combinations thereof.

8. The use according to claim 7, wherein the drug, the preparation or the device is administered in combination with other drugs in the same period, such as simultaneously or successively administered in a specific administration (treatment) process, administered on the same day, administered in the same week, administered in the same month and administered in the same year; or is alternately administered at intervals, such as at an interval of 4 hours, or at an interval of 12 hours, or alternately administered every other day, alternately administered every other week, alternately administered every other month, alternately administered every other year.

9. The use according to one of claims 1-8, wherein the drug, the preparation or the device also comprises other ophthalmic preparations or drugs, including but not limited to drugs for treating myopia, M receptor blockers (such as M3 receptor blockers or antagonists or inhibitors, and atropine), dibazole, polyunsaturated fatty acids (such as DHA, and eicosapentaenoic acid (EPA)), salidroside, prazosin, homatropine, anisodamine (racemic), topicamide, 7-methyl xanthine, nicotinic acid, Piracetam, a red sage root extract, a safflower extract, fish oil, a bear bile extract, vitamins, adenosine triphosphate (ATP), and therapeutic components and adjuvants for ophthalmic diseases.

10. The use according to one of claims 1 to 9, wherein the drug or the preparation is adopted in combination with a device and/or a surgery (such as an orthokeratology, a refractive correction surgery, a myopia corneal laser surgery, and a crystalline lens surgery).

11. The use according to one of claims 1-10, wherein the ω-3 polyunsaturated fatty acid is DHA alone or a composition of DHA and EPA.

12. The use according to claim 11, wherein the daily dose of DHA is in a range of 1 ng-30 g, preferably1 µg-650 mg.

13. The use according to claim 11, wherein a mass ratio of DHA to EPA in the composition of DHA and EPA (DHA: EPA or EPA: DHA) is 2000:1-1:2000, preferably, 500:1-1:500, more preferably, 50:1-1:3; or
in the composition of DHA and EPA, EPA is a predominant component, i.e., EPA: DHA > 1: 1, such as EPA:DHA = 5:1 to EPA:DHA = 99:1; preferably, EPA accounts for: 50% < EPA < 100%; preferably, EPA:DHA = 5:1 to EPA:DHA = 19:1; more preferably, EPA: DHA = 5:1 to EPA:DHA = 9:1; further preferably, EPA:DHA = 9:1, or EPA: DHA=5:1.

14. Use of a scleral lipid metabolism regulating substance as a drug, a preparation or a device for inhibiting negative refraction, ocular axis elongation and/or vitreous chamber elongation of eyes.

15. The use according to claim 14, wherein an individual with ocular axis elongation and/or vitreous chamber elongation refers to an individual with myopia or with a tendency to develop myopia; preferably, the individual with myopia or with a tendency to develop myopia refers to a child and/or an adolescent preferably aged 3-30 years old.

16. The use according to claim 14 or 15, wherein the scleral lipid metabolism regulating substance is an ω-3 polyunsaturated fatty acid, an SREBP-1c inhibitor, and/or a carnitine palmitoyltransferase 2 agonist.

17. The use according to one of claims 14-16, wherein the device is an instrument, equipment, a consumable, a system, a medical device, a health care product or an eye appearance-altering product that can release a drug or has a drug delivery function or has a potential of delivering drugs, such as a corneal contact lens, eyeglasses, an intraocular lense, a suture, an OK mirror cleaning (maintenance) system, an eye patch, an eyesight improving patch, a cosmetic contact lens, a microneedle, an eye spray system, an eye massager, an eye fumigator, an eye surface drug delivery device, an intraocular drug delivery device, a fundus drug delivery device, an implant pump and a wearable device; or
the device is various glasses, corneal contact lenses (OK mirror, etc.) and frame glasses that can delay the progression of myopia, as well as instruments, equipment, consumables, medical instruments or health care products that have a vision protecting function or a myopia treatment (correction) effect, such as eye patches, (myopia) acupoint massagers, eye relaxation equipment and myopia therapeutic equipment.

18. The use according to one of claims 14-17, wherein the preparation is an oral product such as a health care product, food, a dietary supplement, a nutritional product and drink, or a cosmetic; wherein, the cosmetic is a combination of one or more of a free solution, an oil-water mixture, a suspension (agent), a liniment, a lotion, a spray, a cream, a drop, an electuary, an ointment, a paste, a pill, a suppository, an emulsion and a patch.

19. The use according to one of claims 14 to 17, wherein the drug or the preparation is a liquid (such as an injection), a tablet, a lyophilized powder injection, a capsule, an effervescent tablet, a chewable tablet, a buccal tablet, a granule, an ointment, a syrup, an oral liquid, an aerosol, a nasal drop, an external preparation, an oral preparation, etc; preferably, the drug or the preparation is an ophthalmic dosage form, including but not limited to an eye drop (an eye solution), an eye ointment, an eye spray, an implant, an ophthalmic gel, an eye patch, an ophthalmic microsphere, an ophthalmic sustained-release preparation, a periocular injection, an intraocular injection; further, the drug or the preparation is a free solution, an oil-water mixture, a suspension (agent), a liniment, a lotion, a cream, a drop, an electuary, a spray, an ointment, a patch, a paste, a pill, a suppository, a powder, an emulsion, and a formulated composition containing cellulose (such as methylcellulose), a dendrimer, a nanomaterial, a sustained-release material, a liposome or a combination thereof.

20. The use according to one of claims 14-19, wherein the drug or the preparation is administered systemically (e.g. orally, and intravenously), and/or topically (by eye drop, intravitreal injection, skin ointment/emulsion periocular application or eye ointment application), and/or parenterally (such as mucosal administration and transdermal administration).

21. The use according to claim 20, wherein the systemic administration dosage form (e.g. an oral tablet) and the topical administration dosage form (e.g. an eye drop) are used simultaneously, or in combination, or alternately, or at intervals, or alone.

22. The use according to one of claims 14 to 21, wherein the drug, the preparation or the device is administered in combination with other drugs, and the other drugs are drugs for preventing or treating myopia (M receptor blockers such as dibazole, polyunsaturated fatty acids, DHA, fish oil, prazosin, salidroside and atropine, 7-methyl xanthine, and a nicotinic acid), vasodilators, smooth muscle relaxers, drugs for preventing vasospasm, drugs for regulating collagen metabolism, Piracetam, antiallergic drugs, liver-protecting drugs, or a combination thereof.

23. The use according to one of claims 14-22, wherein the drug, the preparation or the device is administered in combination with other drugs in the same period, such as simultaneously or successively administered in a specific administration (treatment) process, administered on the same day, administered in the same week, administered in the same month and administered in the same year; or is alternately administered at intervals, such as at an interval of 4 hours, or at an interval of 12 hours, or alternately administered every other day, alternately administered every other week, alternately administered every other month, alternately administered every other year.

24. The use according to one of claims 14-23, wherein the drug, the preparation or the device also comprises other ophthalmic preparations or drugs, including but not limited to drugs for treating myopia, M receptor blockers (such as M3 receptor blockers or antagonists or inhibitors, atropine), dibazole, polyunsaturated fatty acids (such as DHA and EPA), salidroside, prazosin, homatropine, anisodamine (racemic), topicamide, 7-methyl xanthine, a nicotinic acid, Piracetam, a red sage root extract, a safflower extract, fish oil, a bear bile extract, vitamins, adenosine triphosphate (ATP), and therapeutic components and adjuvants for ophthalmic diseases.

25. The use according to one of claims 14-24, wherein the drug or the preparation is adopted in combination with a device and/or a surgery, such as an orthokeratology, a refractive correction surgery, a myopia corneal laser surgery, and a crystalline lens surgery.

26. The use according to one of claims 14-25, wherein the ω-3 polyunsaturated fatty acid is DHA or a composition of DHA and EPA.

27. The use according to claim 26, wherein the daily dose of DHA is in a range of 1 ng-30 g, preferably1 µg-650 mg.

28. The use according to claim 26, wherein a mass ratio of DHA to EPA in the composition of DHA and EPA (DHA: EPA or EPA: DHA) is 2000:1-1:2000, preferably, 500:1-1:500, more preferably, 50:1-1:3; or
in the composition of DHA and EPA, EPA is a predominant component, i.e., EPA:DHA > 1:1, such as EPA:DHA = 5:1 to EPA:DHA = 99:1; preferably, EPA accounts for: 50% < EPA < 100%; preferably, EPA:DHA = 5:1 to EPA:DHA = 19:1; more preferably, EPA:DHA = 5:1 to EPA:DHA = 9:1; further preferably, EPA:DHA = 9:1, or EPA:DHA=5:1.

29. Use of a substance for regulating eye scleral lipid metabolism in the manufacture of a drug, a preparation or a device for preventing, delaying, inhibiting and/or treating myopia and myopia-related diseases; preferably, the regulation refers to restoring the eye scleral lipid metabolism to be normal, or partially or basically restoring the level of the scleral lipid metabolism to be normal.

30. The use according to claim 29, wherein the eye scleral lipid metabolism regulating substance is a ω-3 polyunsaturated fatty acid, an SREBP-1c inhibitor and/or a carnitine palmitoyltransferase 2 agonist.

31. The use according to claim 29 or 30, wherein the device is an instrument, equipment, a consumable, a system, a medical device, a health care product or an eye appearance-altering product that can release a drug or has a drug delivery function or has a potential of delivering drugs, such as a corneal contact lens, eyeglasses, an intraocular lense, a suture, an OK mirror cleaning (maintenance) system, an eye patch, an eyesight improving patch, a cosmetic contact lens, a microneedle, an eye spray system, an eye massager, an eye fumigator, an eye surface drug delivery device, an intraocular drug delivery device, a fundus drug delivery device, an implant pump and wearable device; or
the device is various glasses, corneal contact lenses (OK mirror, etc.) and frame glasses that can delay the progression of myopia, as well as instruments, equipment, consumables, medical instruments or health care products that have a vision protecting function or a myopia treatment (correction) effect, such as eye patches, (myopia) acupoint massagers, eye relaxation equipment and myopia therapeutic equipment.

32. The use according to one of claims 29-31, wherein the preparation is an oral product such as a health care product, food, a dietary supplement, a nutritional product and drink, or a cosmetic; wherein, the cosmetic is a combination of one or more of a free solution, an oil-water mixture, a suspension (agent), a liniment, a lotion, a spray, a cream, a drop, an electuary, an ointment, a paste, a pill, a suppository, an emulsion and a patch.

33. The use according to one of claims 29-31, wherein the drug or the preparation is a liquid (such as an injection), a tablet, a lyophilized powder injection, a capsule, an effervescent tablet, a chewable tablet, a buccal tablet, a granule, an ointment, a syrup, an oral liquid, an aerosol, a nasal drop, an external preparation, an oral preparation, etc; preferably, the drug or the preparation is an ophthalmic dosage form, including but not limited to an eye drop (an eye solution), an eye ointment, an eye spray, an implant, an ophthalmic gel, an eye patch, an ophthalmic microsphere, an ophthalmic sustained-release preparation, a periocular injection, an intraocular injection; further, the drug or the preparation is a free solution, an oil-water mixture, a suspension (agent), a liniment, a lotion, a cream, a drop, an electuary, a spray, an ointment, a patch, a paste, a pill, a suppository, a powder, an emulsion, and a formulated composition containing cellulose (such as methylcellulose), a dendrimer, a nanomaterial, a sustained-release material, a liposome or a combination thereof.

34. The use according to one of claims 29-33, wherein the drug or the preparation is administered systemically (e.g., orally and intravenously), and/or topically (by dropping into eyes, intravitreal injection, skin ointment/emulsion periocular application or eye ointment application), and/or parenterally (such as mucosal administration and transdermal administration).

35. The use according to claim 34, wherein the systemic administration dosage form (e.g. an oral tablet) and the topical administration dosage form (e.g. an eye drop) are used simultaneously, or in combination, or alternately, or at intervals, or alone.

36. The use according to one of claims 29-35, wherein the drug, preparation or device is administered in combination with other drugs, and the other drugs are drugs for preventing or treating myopia (M receptor blockers such as dibazole, polyunsaturated fatty acids, DHA, fish oil, prazosin, salidroside and atropine, 7-methyl xanthine, and a nicotinic acid), vasodilators, smooth muscle relaxers, drugs for preventing vasospasm, drugs for regulating collagen metabolism, Piracetam, antiallergic drugs, liver-protecting drugs, or combinations thereof.

37. The use according to claim 36, wherein the drug, preparation or device is administered in combination with other drugs in the same period, such as simultaneously or successively administered in a specific administration (treatment) process, administered on the same day, administered in the same week, administered in the same month and administered in the same year; or is alternately administered at intervals, such as at an interval of 4 hours, or at an interval of 12 hours, or alternately administered every other day, alternately administered every other week, alternately administered every other month, alternately administered every other year.

38. The use according to one of claims 29-37, wherein the drug, preparation or device also comprises other ophthalmic preparations or drugs, including but not limited to drugs for treating myopia, M receptor blockers (such as M3 receptor blockers or antagonists or inhibitors, atropine), dibazole, polyunsaturated fatty acids (such as DHA and EPA), salidroside, prazosin, homatropine, anisodamine (racemic), topicamide, 7-methyl xanthine, a nicotinic acid, Piracetam, a red sage root extract, a safflower extract, fish oil, a bear bile extract, vitamins, adenosine triphosphate (ATP), and therapeutic components and adjuvants for ophthalmic diseases.

39. The use according to one of claims 29-38, wherein the drug or the preparation is adopted in combination with a device and/or a surgery, such as an orthokeratology, a refractive correction surgery, a myopia corneal laser surgery, and a crystalline lens surgery.

40. The use according to one of claims 29-39, wherein the ω-3 polyunsaturated fatty acid is DHA or a composition of DHA and EPA.

41. The use according to claim 40, wherein the daily dose of DHA in a range of 1 ng-30 g, preferably1 µg-650 mg.

42. The use according to claim 40, wherein a mass ratio of DHA to EPA in the composition of DHA and EPA (DHA: EPA or EPA: DHA) is 2000:1-1:2000, preferably, 500:1-1:500, more preferably, 50:1-1:3; or
in the composition of DHA and EPA, EPA is a predominant component, i.e., EPA:DHA > 1: 1, such as EPA:DHA = 5:1 to EPA:DHA = 99:1; preferably, EPA accounts for: 50% < EPA < 100%; preferably, EPA:DHA = 5:1 to EPA:DHA = 19:1; more preferably, EPA: DHA = 5:1 to EPA:DHA = 9:1; further preferably, EPA:DHA = 9:1, or EPA: DHA=5:1.

43. The use according to one of claims 29-42, wherein the myopia is refractive myopia and/or axial myopia; congenital myopia (myopia at birth or before school age), early-onset myopia (under 14 years old), delayed myopia (16-18 years old), late-onset myopia (after adulthood); low myopia (mild myopia), moderate myopia, high myopia (severe myopia); pseudomyopia, true myopia; childhood and/or adolescent myopia (preferably 3-26 years old, more preferably 6-18 years old), myopia in minors, myopia in adults, and myopia in the elderly; simple myopia, and pathological myopia; axial simple myopia, and simple axial myopia; axial myopia in children and/or adolescents (preferably 3-26 years old, more preferably 6-18 years old); axial myopia in school-aged and preschool-aged populations; primary myopia, and secondary myopia; primary myopia in children and/or adolescents (preferably 3-26 years old, more preferably 6-18 years old); or progressive myopia in children and/or adolescents (preferably 3-26 years old, more preferably 6-18 years old).

44. The use according to one of claims 29-43, wherein people involved in the myopia refer to children and/or adolescents, preferably people aged 3-26 years, more preferably people aged 6-18 years; or refer to minors, preferably people whose eyes (eyeballs) are still in the stage of growth and development.

45. The use according to one of claims 29-44, wherein the myopia-related diseases comprise: vitreous opacity, retinal hemorrhage and detachment caused by myopia, complications of high myopia such as floaters, glaucoma, posterior staphylomas, retinal detachment, retinal tear, amblyopia, choroidal neovascularization, macular hemorrhage, choroidal atrophy, macular degeneration or denaturation, visual field defect, progressive or sudden decline of vision (especially near vision), eye soreness and/or pain, and night blindness.

46. The use according to one of claims 29-45, wherein the eye scleral lipid metabolism regulating substance comprises a derivative of ω-3 polyunsaturated fatty acid.

47. The use according to claim 46, wherein the derivative of ω-3 polyunsaturated fatty acid comprises an ester of ω-3 polyunsaturated fatty acid.

48. The use according to one of claims 29-47, wherein the drug further contains a pharmaceutically acceptable adjuvant.

49. The use according to one of claims 29-48, the lipid in wherein eye scleral lipid metabolism comprises simple lipids, compound lipids and derivatives thereof, such as DHA and/or EPA.

50. An eye drop, an ophthalmic gel or an ocular injection for prevention, inhibition, alleviation or treatment of myopia, wherein the active ingredient in the eye drop, the ophthalmic gel or the ocular injection is an eye scleral lipid metabolism regulator.

51. The eye drop, the ophthalmic gel or the ocular injection according to claim 50, wherein the regulator for the eye scleral lipid metabolism is an SREBP-1c inhibitor, a carnitine palmitoyltransferase 2 agonist and/or a ω-3 polyunsaturated fatty acid.

52. The eye drop, the ophthalmic gel or the ocular injection according to claim 51, wherein the ω-3 polyunsaturated fatty acid is DHA, or a composition of DHA and EPA.

53. The eye drop, ophthalmic gel or ocular injection according to claim 52, wherein the daily dose of DHA is 1 ng-30 g, preferably1 µg-650 mg.

54. The eye drop, the ophthalmic gel or the ocular injection according to claim 52, wherein a mass ratio of DHA to EPA in the composition of DHA and EPA (DHA:EPA or EPA:DHA) is 2000:1-1:2000, preferably, 500:1-1:500, more preferably, 50:1-1:3; or
in the composition of DHA and EPA, EPA is a predominant component, i.e., EPA: DHA > 1: 1, such as EPA:DHA = 5:1 to EPA:DHA = 99:1; preferably, EPA accounts for: 50% < EPA < 100%; preferably, EPA:DHA = 5:1 to EPA:DHA = 19:1; more preferably, EPA: DHA = 5:1 to EPA: DHA = 9:1; further preferably, EPA:DHA = 9:1, or EPA:DHA=5:1.

55. The eye drop, the ophthalmic gel or the ocular injection according to one of claims 50-54, wherein the eye drop, the ophthalmic gel or the ocular injection further contains a pharmaceutically acceptable adjuvant.

56. The eye drop, ophthalmic gel or ocular injection according to claim 55, wherein the pharmaceutically acceptable adjuvant is selected from an excipient, a disintegrant, an osmotic pressure regulator, a PH modifier, a viscosity modifier, a solubilizer, a stabilizer, a bacteriostatic agent and/or an antioxidant.

57. The eye drop, the ophthalmic gel or the ocular injection according to claim 56, wherein the pH modifier is selected from a boric acid buffer and a phosphate buffer; or the osmotic pressure regulator is sodium chloride, boric acid or borax; or the bacteriostatic agent is selected from phenylmercuric nitrate and phenylmercuric acetate; or, the viscosity modifier is selected from methyl cellulose, polyethylene glycol (PEG), polyethylene, and povidone; or the antioxidant is vitamin E.

58. A drug, preparation or device for regulating eye scleral lipid metabolism, comprising an SREBP-1c inhibitor, a carnitine palmitoyltransferase 2 agonist and/or a ω-3 polyunsaturated fatty acid.

59. The drug, the preparation or the device according to claim 58, wherein the device is an instrument, equipment, consumable, system, medical device, health care product or eye appearance-altering product that can release a drug or has a drug delivery function or has a potential of delivering drugs, such as a corneal contact lens, eyeglasses, an intraocular lens, a suture, an OK mirror cleaning (maintenance) system, an eye patch, an eyesight improving patch, a cosmetic contact lens, a microneedle, an eye spray system, an eye massager, an eye fumigator, an eye surface drug delivery device, an intraocular drug delivery device, a fundus drug delivery device, an implant pump and a wearable device; or
the device is various glasses, corneal contact lenses (OK mirror, etc.) and frame glasses that can delay the progression of myopia, as well as instruments, equipment, consumables, medical instruments or health care products that have a vision protecting function or a myopia treatment (correction) effect, such as eye patches, (myopia) acupoint massagers, eye relaxation equipment and myopia therapeutic equipment.

60. The drug, preparation or device according to claim 58 or 59, wherein the preparation is an oral product such as a health care product, food, a dietary supplement, a nutritional product and drink, or a cosmetic; wherein, the cosmetic is a combination of one or more of a free solution, an oil-water mixture, a suspension (agent), a liniment, a lotion, a spray, a cream, a drop, an electuary, an ointment, a paste, a pill, a suppository, an emulsion and a patch.

61. The drug, preparation or device according to claim 58 or 59, wherein the drug or the preparation is a liquid (such as an injection), a tablet, a lyophilized powder injection, a capsule, an effervescent tablet, a chewable tablet, a buccal tablet, a granule, an ointment, a syrup, an oral liquid, an aerosol, a nasal drop, an external preparation, an oral preparation, etc; preferably, the drug or the preparation is an ophthalmic dosage form, including but not limited to an eye drop (an eye solution), an eye ointment, an eye spray, an implant, an ophthalmic gel, an eye patch, an ophthalmic a microsphere, an ophthalmic sustained-release preparation, a periocular injection, and an intraocular injection; further, the drug or the preparation is a free solution, an oil-water mixture, a suspension (agent), a liniment, a lotion, a cream, a drop, an electuary, a spray, an ointment, a patch, a paste, a pill, a suppository, a powder, an emulsion, and a formulated composition containing cellulose (such as methylcellulose), a dendrimer, a nanomaterial, a sustained-release material, a liposome or a combination thereof.

62. The drug, preparation or device according to one of claims 58-61, wherein the drug, the preparation or the device also comprises other ophthalmic preparations or drugs, including but not limited to drugs for treating myopia, M receptor blockers (such as M3 receptor blockers or antagonists or inhibitors, atropine), dibazole, polyunsaturated fatty acids (such as DHA, and EPA), salidroside, prazosin, homatropine, anisodamine (racemic), topicamide, 7-methyl xanthine, a nicotinic acid, Piracetam, a red sage root extract, a safflower extract, fish oil, a bear bile extract, vitamins, adenosine triphosphate (ATP), and therapeutic components and adjuvants for ophthalmic diseases.

63. The drug, preparation or device according to one of claims 58-62, wherein the ω-3 polyunsaturated fatty acid is DHA, or a composition of DHA and EPA.

64. The drug, preparation or device according to claim 63, wherein the daily dose of DHA is in a range of 1 ng-30 g, preferably1 µg-650 mg.

65. The drug, preparation or device according to claim 63, wherein a mass ratio of DHA to EPA in the composition of DHA and EPA (DHA:EPA or EPA:DHA) is 2000:1-1:2000, preferably, 500:1-1:500, more preferably, 50:1-1:3; or
in the composition of DHA and EPA, EPA is a predominant component, i.e., EPA: DHA > 1: 1, such as EPA:DHA = 5:1 to EPA:DHA = 99:1; preferably, EPA accounts for: 50% < EPA < 100%; preferably, EPA:DHA = 5:1 to EPA:DHA = 19:1; more preferably, EPA:DHA = 5:1 to EPA:DHA = 9: 1; further preferably, EPA:DHA = 9:1, or EPA:DHA=5:1.

66. The drug, preparation or device according to one of claims 58 to 65, wherein the drug, the preparation or the device further contains a pharmaceutically acceptable adjuvant.

67. A method for improving the blood perfusion (blood flow) of ocular choroid in an individual, or a method for increasing the thickness of ocular choroid in an individual, or a method for inhibiting the increase of HIF-1 α protein level of eyes in an individual, or a method for increasing the expression of carnitine palmitoyltransferase 2 (Cpt2) of eyes in an individual, wherein, an ω-3 polyunsaturated fatty acid is administered to the individual.

68. The method according to claim 67, wherein the individual is a person with myopia or with a tendency to develop myopia, preferably a person whose eyes are in growth and development, more preferably a school-aged person.

69. The method according to claim 67 or 68, wherein the ω-3 polyunsaturated fatty acid is DHA or a composition of DHA and EPA.

70. The method according to claim 69, wherein the daily dose of DHA is in a range of 1 ng-30 g, preferably1 µg-650 mg.

71. The method according to claim 69, wherein a mass ratio of DHA to EPA in the composition of DHA and EPA (DHA:EPA or EPA:DHA) is 2000:1-1:2000, preferably, 500:1-1:500, more preferably, 50:1-1:3; or in the composition of DHA and EPA, EPA is a predominant component, i.e., EPA:DHA > 1: 1, such as EPA:DHA = 5:1 to EPA:DHA = 99:1; preferably, EPA accounts for: 50% < EPA < 100%; preferably, EPA:DHA = 5:1 to EPA: DHA = 19:1; more preferably, EPA:DHA = 5:1 to EPA:DHA = 9:1; further preferably, EPA:DHA = 9:1, or EPA:DHA=5:1.

72. A new target or new mechanism for prevention and control of myopia in an individual, wherein an individual who is prone to myopia, has myopia or has a tendency to be myopia , has scleral lipid metabolism disorder, and the purpose of preventing and controlling myopia is achieved by regulating the level of the scleral lipid metabolism.
